# EUROPEAN PATENT APPLICATION

(11) **EP 1 790 706 A1**
(43) Date of publication of application: **30.05.2007**
(21) Application number: 05748965.0
(22) Date of filing: 13.06.2005
(51) Int. Cl.: C09K 11/08

(54) **FLUORESCENT MATERIAL, FLUORESCENT MATERIAL COMPOSITION AND METHOD OF FLUORESCENT DETECTION**

(30) Priority: 22.06.2004 JP 2004183512
(71) Applicant: FUJIFILM Corporation, Minato-ku Tokyo (JP)
(72) Inventor: NISHIGAKI, Junji, c/o FujiFilm Corporation, Minami-Ashigara-shi, Kanagawa 2500123 (JP); KOJIMA, Masayoshi, c/o FujiFilm Corporation, Minami-Ashigara-shi, Kanagawa 2500123 (JP); HIRAI, Hiroyuki, c/o FujiFilm Corporation, Minami-Ashigara-shi, Kanagawa 2500123 (JP)
(74) Representative: HOFFMANN EITLE
(86) International application number: PCT/JP2005/010767
(87) International publication number: WO 2005/123874

(57) **Abstract**

Two or more target substances are simply and efficiently detected using one type of nanoparticle.

Two or more target substances contained in one sample are labeled with two or more fluorescent dyes each capable of binding to the respective target substance and each of which emit light at wavelengths different from each other, combination is made with a phosphor nano-particle capable of binding to each of the two or more fluorescent dyes, subsequently irradiation is undertaken with an excitation light which excites the phosphor nano-particle to emit light, and the two or more fluorescent dyes are caused to emit fluorescence using the light from the phosphor nano-particle as an excitation light.

## Description

### Technical Field

The invention relates to a fluorescent material, a fluorescent composition, and a fluorescence detection method, and specifically to a fluorescent material and a fluorescent composition containing a phosphor nano-particle, and a fluorescence detection method using the same.

### Background Art

It is known that particle materials of nano size exhibit properties different from that of the bulk materials. For example, with a nano-scale semiconductor, the band gap, which has conventionally been considered to be material-specific, varies depending upon the size of the particle, which is well known as the so-called quantum size effect. The particle size at which the effect is significant is generally several tens of nm or less, depending on the type of semiconductor material. Thus, single order nanoparticles are particularly important. Some materials are also known in which, as the quantum size effect becomes significant, the fluorescence lifetime becomes shorter, a certain luminescence previously not observed is observed, and other effects. As mentioned above, nanosized materials, in particular single order nanosized materials, can exhibit different properties from known bulk materials, and thus are attracting widespread attention in science and engineering.

For example, a semiconductor phosphor nano-particle with which semiconductor nanoparticles such as CdSe/CdS (core/shell) and CdSe/ZnS (core/shell) are used and a molecular probe is bound to the surface of beads containing these semiconductor nanoparticles for detection of target molecules has been proposed (for example, see Non-patent Documents 1 and 2). Emission at different wavelengths can be obtained using these semiconductor nanoparticles by varying the crystallite size. If the labeled beads are encoded with combinations of luminescence wavelength and luminescence intensity, simultaneous multiple measurements are possible. Semiconductor phosphor nano-particles have excellent properties for labeling materials, such as high sensitivity, low cost, and ease of automation. Accordingly, the use of the semiconductor phosphor nano-particle as labeling materials has allowed highly-sensitive and high-speed detection of specific site of organisms, certain substances in blood plasma, and the like.

Another semiconductor phosphor nano-particle is proposed wherein the surface of a semiconductor nanoparticle is coated with a modifying molecule to improve the affinity to a matrix (for example, see Patent Documents 1-3, Non-patent Document 3). By coating the semiconductor nanoparticle with the modifying molecule, it becomes possible, for example, to improve the affinity to an aqueous medium, and the dispersibility in organic polymers and organic solvents. Thus, it facilitates application of semiconductor phosphor nano-particles as labeling materials. As described above, semiconductor phosphor nano-particles are expected to be widely applicable in fields such as clinical diagnosis and biochemical and medical science research.

In the fields of biochemical research and medical science research, exact detection of complicated vital phenomena is required. Simultaneous detection of plural substance in a sample may be required, for example, when the interaction between two or more types of substances is being detected, or when necessary samples cannot be prepared in large amounts. For conducting such tests, a detection system has been developed that uses plural phosphor nano-particles. In particular, in order to avoid enlargement and complexity of equipment due to using excitation light sources corresponding to various fluorescent materials, a system utilizing the size effect of semiconductor phosphor nano-particles was developed (for example, see Patent Document 4). In such a system, excitation filters corresponding to the number of fluorescent material types of different sizes are provided for one excitation light source, and light emitted by the excitation light source is, by using the light source excitation filters, made into a spectrum of excitation light suitable for each fluorescent material, and irradiated.

However, in a system in which the size effect of phosphor nano-particles is utilized, it is necessary to prepare phosphor nano-particles of different sizes.
Furthermore, installation of excitation filters corresponding to plural phosphor nano-particles makes the equipment configuration complicated and simple detection cannot be carried out.

Patent Document 1: U.S. Patent No. 6,319,426
Patent Document 2: Japanese Patent Application Laid-Open (JP-A) No. 2002-38145
Patent Document 3: JP-A No. 2003-64278
Patent Document 4: JP-A No. 2002-28797
Non-patent Document 1: Science, Vol. 281, No. 25, 1998, pp. 2013-2016
Non-patent Document 2: Nature Biotechnology, Vol. 19, 2001, pp. 631-6354
Non-patent document 3: Science, Vol. 281, No. 25, 1998, pp. 2016-2018

### Disclosure of Invention

### Problem to be Solved by the Invention

Accordingly, the object of the present invention is to provide a fluorescent material, a fluorescent composition, and a fluorescence detection method which allow the simple and efficient detection of two or more target substances by using one type of nanoparticle.

### Means for Solving the Problem

The fluorescent material of the present invention comprises two or more fluorescent dyes that emit light at wavelengths different from each other, and a phosphor nano-particle bound to each of the two or more fluorescent dyes.
The phosphor nano-particles are preferably capable of transferring energy to the fluorescent dye at energy levels different from each other.
The fluorescent composition of the present invention comprises two or more fluorescent dyes that emit light at wavelengths different from each other, and a phosphor nano-particle capable of binding to each of the two or more fluorescent dyes.

Furthermore, the fluorescence detection method of the present invention is a fluorescence detection method for detecting two or more target substances contained in one sample by using two or more fluorescent dyes, each of the fluorescent dyes being capable of binding to the respective target substance and each of the fluorescent dyes emitting light at a wavelength different from each other, the method including: forming a complex in which a phosphor nano-particle capable of binding to the two or more fluorescent dyes, the fluorescent dyes, and the target substances are bound together; irradiating an excitation light for exciting the phosphor nano-particle to cause the phosphor nano-particle to emit light; exciting the two or more fluorescent dyes bound to the phosphor nano-particle with light emitted from the phosphor nano-particle to cause the fluorescent dyes to emit fluorescence; and detecting fluorescence emitted from the fluorescent dyes.

### Effect of the Invention

According to the present invention, since one phosphor nano-particle is bound to two or more fluorescent dyes which emit light at wavelengths different from each other, once the phosphor nano-particle is excited by a single excitation light, fluorescence emission from the phosphor nano-particle can cause the two or more fluorescent dyes bound to the phosphor nano-particle to emit light. As a result, plural target substances can be simply and efficiently detected by using one type of phosphor nano-particle without irradiating plural excitation lights, and without using plural nanoparticles.

### Brief Description of the Drawings

Fig. 1 shows a fluorescence spectrum of a phosphor nano-particle in an embodiment of the invention;
Fig. 2 shows a fluorescent material spectrum of a fluorescent material constituted by various fluorescent dyes and a phosphor nano-particle in an embodiment of the invention.

### Best Mode for Carrying Out the Invention

The fluorescent material of the invention comprises two or more fluorescent dyes that emit light at wavelengths different from each other, and a phosphor nano-particle bound to each of the two or more fluorescent dyes. The fluorescent composition of the invention comprises two or more fluorescent dyes that emit light at wavelengths different from each other, and a phosphor nano-particle which is capable of binding to the two or more fluorescent dyes.

In the fluorescent material and fluorescent composition, once the phosphor nano-particle is excited when the phosphor nano-particle is bound to the fluorescent dyes, the phosphor nano-particle emits light, and energy is transmitted from the phosphor nano-particle to the fluorescent dyes. As a result, the fluorescent dyes provided with energy from the phosphor nano-particle are excited to emit light at wavelengths different from each other. Accordingly, when two or more target substances are labeled with the fluorescent dyes, plural target substances can be detected by exciting the phosphor nano-particle with a single excitation light. Furthermore, when two or more fluorescent dyes whose absorption spectrum maxima are close to their fluorescence spectrum maxima , energy transfer occurs even between the two or more fluorescent dyes excited by the phosphor nano-particle, by which exclusively the fluorescent dye having the longest wavelength can be caused to emit light. In such a case, sequential energy transfer is inhibited if at least one of the target substances labeled with a fluorescent dye is absent. Therefore, the absence of the target substance can be detected from a change of the light emitting behavior.
In the invention, the expression "fluorescent composition" includes a state in which the phosphor nano-particle and fluorescent dyes are bound to each other, and a state in which the phosphor nano-particle and fluorescent dyes are present but not bound together.
The invention is further described below in detail.

### [1] Phosphor nano-particle

The phosphor nano-particle in the invention is a particle which is capable of emitting fluorescence by a predetermined excitation light and is capable of binding the below-mentioned fluorescent dyes to at least a portion of the surface thereof.

The phosphor nano-particle includes any particles as long as they have "nanosized" number average particle diameter. The number average particle diameter is preferably 0.5 to 100 nm, more preferably 0.5 to 50 nm, and further preferably 1 to 10 nm. The particle diameter distribution of the fluorescent material nanoparticle is a coefficient of variation preferably 0 to 50%, more preferably 0 to 20%, and further preferably 0 to 10%. The coefficient of variation means the arithmetic standard deviation divided by the number average particle diameter expressed in terms of a percentage ((arithmetic standard deviation x 100) / number average particle diameter).

The phosphor nano-particle is preferably a phosphor nano-particle of a metal oxide or a metal sulfide. Examples of the metals constituting a metal oxide or a metal sulfide include group IIB metals such as Zn, group IIIA metals such as Y, Eu, and Tb, group IIIB metals such as Ga and In, group IVA metals such as Zr and Hf, group IVB metals such as Si and Ge, group VA metals such as V and Nb, and group VIA metals such as Mo and W. Among them, Zn, which exhibits no high reactivity to organisms, is particularly preferable. Furthermore, the metal may be a composite metal oxide such as Zn₂SiO₄, CaSiO₃, MgWO₄, YVO₄, and Y₂SiO₅. The phosphor nano-particle in the invention is particularly preferably zinc oxide (ZnO) or zinc sulfide (ZnS), and most preferably zinc oxide (ZnO) from the viewpoints of stable production, few concerns about toxicity, low cost production thereof, high monodispersibility of particles, strong light emitting capability, and ease of matching of the wavelength range of the emission spectrum with the present object.

Furthermore, the phosphor nano-particle of a metal oxide or a metal sulfide preferably contains a small amount of a metal ion different from the metal contained in the constituent metal oxide or metal sulfide. Examples of such a metal ion include metal ions such as Mn, Cu, Eu, Tb, Tm, Ce, A1, and Ag. Among them, Mn and Eu are preferable from the viewpoints of high visibility of light emission and stable production. These metal ions are also preferably doped as a compound combined with a chloride ion or a fluoride ion. The metal ions to be doped may comprise one type of atom, or plural types of atoms. Accordingly, examples of the phosphor nano-particle comprising these metal ions include ZnS/Mn and ZnO/Eu and the like. The concentration of the metal ion varies in optimum amount, depending upon the metal constituting the phosphor nano-particle and the type thereof, but preferably in the range of 0.001 to 10 atom %, and more preferably in the range of 0.01 to 10 atom %.

The phosphor nano-particle according to the invention is preferably excited with a light in the ultraviolet region, more preferably 250 nm to 380 nm, and emits a visible light, more preferably 400 nm to 700 nm, from the viewpoints of separation between the excitation light and the signal fluorescence, the utilization of a low-cost light source, and construction of a simple detection system. The emission of a visible light allows excitation of fluorescent dyes in the visible range, which can be caused to display color at lower energy and with no reactivity, particularly no severe reactivity to organisms.

The phosphor nano-particle in the invention is preferably a particle capable of transferring energy to the fluorescent dyes bound to the surface of the material with energy levels different from each other. By doing so the phosphor nano-particle can cause the fluorescent dyes excited at different energy levels to excite at the same time.
The half width for the light emission of the phosphor nano-particle is preferably 50 to 200 nm, and in order to detect light emission with high sensitivity with a simple apparatus, it is preferably 60 to 180 nm. Furthermore, as a fluorescent labeling material, it is preferable that the light emission peak wavelength and absorption peak wavelength thereof are different from each other. The emission peak wavelength is more preferably distanced by 20 nm or more, most preferably distanced by 50 nm or more from the edge of the absorption wavelength for the highly sensitive detection of light emission. Phosphor nano-particles having such emission peak wavelengths and half widths are phosphor nano-particle of a metal oxide or a metal sulfide, and can be readily obtained by those skilled in the art by appropriately selecting the constituent metal or the like as mentioned above.

Furthermore, the phosphor nano-particle of a metal oxide or a metal sulfide of the invention is preferably a phosphor nano-particle of a metal oxide having excellent coatability with the surface modifying agent later described.

### [2] Surface modifying agent

When the phosphor nano-particle in the invention is a phosphor nano-particle of a metal oxide or a metal sulfide, it is particularly preferable that it is surface-modified by using a compound represented by the following formula (I) or a decomposition product thereof as a surface modifying agent. The dispersibility of the phosphor nano-particle in water or a hydrophilic solvent can be improved, and the elution of the phosphor nano-particle or quenching of fluorescence by the body fluid or the like can be prevented and, therefore, a high sensitivity can be provided. Furthermore, the modified material is advantageous in that it has a uniform emission characteristic with a broad half width, and is readily imparted with functions such as binding to molecule probes for detecting target molecules.

M-(R)₄ formula (I)

In the formula, M represents a Si or Ti atom, and R represents an organic group. Each R group may be the same or different from each other, but at least one R group represents a group having reactivity to the affinity molecule.

Among the organic groups represented by R, examples of groups having reactivity with an affinity molecule include a group having, at a terminal, a vinyl group, an allyloxy group, an acryloyl group, a methacryloyl group, an isocyanato group, a formyl group, an epoxy group, a maleimide group, a mercapto group, an amino group, a carboxyl group, a halogen, or the like, the group being connected through a linking group L. Among the groups having reactivity with an affinity molecule, those having an amino group at the terminal thereof are particularly preferable.

Examples of the linkage group L include alkylene groups (for example, linear or cyclic groups having 1 to 10 carbon atoms, preferably having 1 to 8 carbon atoms, such as a methylene group, an ethylene group, a trimethylene group, a tetramethylene group, a hexamethylene group, a propylene group, an ethylethylene group, and a cyclohexylene group).
The linking group L may have an unsaturated bond. Examples of unsaturated groups include alkenylene groups (linear or cyclic groups having 1 to 10 carbon atoms, preferably having 1 to 8 carbon atoms, such as a vinylene group, a propenylene group, 1-butenylene group, 2-butenylene group, 2-pentenylene group, 8-hexadecenylene group, 1,3-butanedienylene group, and cyclohexenylene group), and arylene groups (arylene groups having 6 to 10 carbon atoms such as a phenylene group and a naphthylene group, preferably a phenylene group having 6 carbon atoms).

The linking group L may have one or more heteroatoms (a heteroatom means any atom other than a carbon atom, such as a nitrogen atom, oxygen atom, or sulfur atom). The heteroatom is preferably an oxygen atom or sulfur atom, and most preferably an oxygen atom. The number of hetero atoms is not particularly limited, but it is preferably five or less, and more preferably three or less.

The linking group L may have, as a partial structure, a functional group containing a carbon atom adjacent to the heteroatom. Examples of the functional group include an ester group (including a carboxylate ester, carbonate ester, sulfonate ester, and sulfinate ester), an amide group (including a carboxylamide, urethane, sulfonamide, and sulfinamide), an ether group, thioether group, a disulfide group, an amino group, and an imide group. The above-described functional groups may further have a substituent. The linking group L may have a plurality of such functional groups. When the linking group L has a plurality of such functional groups, they may be the same or different from each other.
The functional group is preferably an ester group, amide group, ether group, thioether group, disulfide group, or amino group, and more preferably an alkenyl group, ester group, or ether group.

The other organic group represented by R may be any group, but preferably an alkoxy group, such as a methoxy group, ethoxy group, isopropoxy group, n-propoxy group, t-butoxy group, and n-butoxy group, or a phenoxy group. These alkoxy group and phenoxy groups may further have a substituent, but it is desirable that the total number of carbon atoms is 8 or less. The surface modifying agent used in the invention may be one in which an amino group, carboxyl group, or the like forms a salt with an acid or base.

The decomposition products of a compound represented by the formula (I) among the surface modifying agents used in the invention refer to hydroxides produced by hydrolysis of an alkoxy group, low molecular weight oligomers produced by dehydration condensation reaction between hydroxyl groups (the oligomers may have any of linear, cyclic, or crosslinking structures) and the like, dealcoholization condensation reaction products of a hydroxyl group and an unhydrolyzed alkoxy group, and sols and gels produced by further dehydration condensation reaction of the above products.

Specific examples of the surface modifying agents used in the invention include the following compounds, but the agents are not limited to these compounds in the invention:
N-(2-aminoethyl)-3-aminopropylmethyldimethoxy silane, N-(2-aminoethyl)-3-aminopropyltrimethoxy silane, N-(2-aminoethyl)-3-aminopropyltriethoxy silane, 3-aminopropyltrimethoxy silane, aminophenyltrimethoxy silane, 3-aminopropyltriethoxy silane, bis(trimethoxysilylpropyl)amine, N-(3-aminopropyl)-benzamidetrimethoxy silane, 3-hydrazidepropyltrimethoxy silane, 3-maleimidepropyltrimethoxy silane, (p-carboxy)phenyltrimethoxy silane, 3-carboxypropyltrimethoxy silane, 3-aminopropyltitaniumtripropoxide, 3-amino propylmethoxyethyltitaniumdiethoxide, and 3-carboxypropyltitaniumtrimethoxide, and the like.

The surface modifying agent used in the invention may be a compound in which an NH₂ group or COOH group at the terminal forms salt with an acid or base.
The surface modifying agent which can be used in the invention may coat the entire surface of the phosphor nano-particle or may be bound to a part thereof. In the invention, the surface modification agent may be used alone or in combination with another.

In the invention, in addition to the above-described surface modifying agents, known surface modification agents (for example, polyethylene glycol, polyoxyethylene(1)lauryl ether phosphate, lauryl ether phosphate, trioctylphosphine, trioctylphosphine oxide, sodium polyphosphate, and sodium bis (2-ethylhexyl) sulfosuccinate) may coexist during or after the synthesis of the nanoparticle.

### [3] Method of preparing phosphor nano-particle

The metal oxide phosphor nano-particle according to the invention can be obtained by a solution-phase synthesis method such as a sol-gel method in which an organic metal compound of alkoxide, acetylacetonate or the like of the metal is hydrolyzed, a hydroxide precipitation method in which an alkaline is added to an aqueous solution of a salt of the metal to precipitate it as a hydroxide followed by dehydration and annealing, an ultrasonic decomposition method in which the above-described precursor solution of the metal is irradiated with ultrasound, a solvothermal method that carries out a decomposition reaction under high temperature and high pressure, and a spray pyrolysis method that carries out spraying at high temperature, or the like. The phosphor nano-particle can also be obtained by a vapor-phase synthesis method such as a thermal CVD method and a plasma CVD method that use an organic metal compound, and a sputtering method that uses a target of a metal or a metal oxide, a laser ablation method, or the like.

The metal sulfide phosphor nano-particle according to the invention can be obtained by a solution-phase synthesis method such as: a hot soap method in which a heat decomposable metal compound such as a diethyl dithiocarbamate compound of the metal is crystal grown in a high boiling point organic solvent such as trialkylphosphineoxides, trialkylphosphines, ω-aminoalkanes; a coprecipitation method in which a solution of a sulfide such as sodium sulfide or ammonium sulfide is added to a solution of a salt of the metal to grow crystals; and a reversed micelle method in which an aqueous solution of the above-described raw materials containing a surfactant is made to exist as a reversed micelle in a nonpolar organic solvent such as alkanes, ethers, and aromatic hydrocarbons and is crystal grown in the reversed micelle. The phosphor nano-particle can also be obtained by one of the vapor-phase synthesis methods described as methods for forming the metal oxide phosphor nano-particle.

The surface modifying agent used in the invention can be added during the synthesis of the phosphor nano-particle, but preferably it is added after the synthesis, and then at least a part thereof is hydrolyzed to bind to the phosphor nano-particle, and causing at least a portion of the surface of the nanoparticle to be coated (surface modified). The phosphor nano-particle may be washed and purified by a normal method, such as centrifuge separation and filtration, and then dispersed in a solvent (preferably a hydrophilic organic solvent such as methanol, ethanol, isopropyl alcohol, and 2-ethoxyethanol) containing the surface modifying agent used in the invention for coating.

The addition amount of the surface modifying agent used in the invention is varied depending on the particle size of the fluorescent material, the concentration of particles, and the type (size and structure and the like) of surface modifying agent, but is preferably 0.001 to 10 mol times, more preferably 0.01 to 2 mol times relative to a metal oxide or a metal sulfide.
In the invention, known surface modifying agents can also be used. The addition amount of the known surface modifying agent is not particularly limited, but is preferably 0.01 to 100 times mol, and more preferably 0.05 to 10 times mol relative to the surface modifying agent represented by the formula (I).

The concentration of the phosphor nano-particle in a dispersion liquid of the phosphor nano-particle bound to the surface modifying agent is not particularly limited because it varies depending on the fluorescence intensity, but is preferably 0.01 mM to 1000 mM, and more preferably 0.1 mM to 100 mM. As the dispersion medium, besides the above-described alcohols, a hydrophilic organic solvent such as DMF, DMSO, and THF and water are preferable.

The fact that the surface of the phosphor nano-particle is coated with the surface modifying agent can be confirmed by chemical analysis and by the presence of a uniform interval between the particles when observed by a high-resolution TEM such as an FE-TEM.

### [4] Fluorescent dye

The fluorescent dyes in the invention are two or more fluorescent dyes excited by light of wavelengths different from each other. The use of the two or more fluorescent dyes allowes the efficient detection of plural target substances corresponding to the fluorescent dyes.
In particular, the fluorescent dyes in the invention are preferably excited by a light in the visible range emitted from the phosphor nano-particle to emit fluorescence in the visible range, and more preferably have a fluorescence spectrum maximum between 400 nm and 800 nm. Examples of such fluorescent dyes include cyanine dyes (for example, Cy3 and Cy5 of CyDye^{™} series), fluorescein dyes, rhodamine dyes, Alexa dye series from Invitrogen Japan K.K., BODIPY dye series, Texas Red dye series , and azacyanine dyes described as Compound Examples I-1 through I-74 in WO 01/021624.

These fluorescent dyes are, as described below, treated so as to be capable of binding with the phosphor nano-particle and target substance (binding between a fluorescent dye and a target substance may be referred to as labeling of a target substance). The labeling of a target substance is similar to the below-mentioned binding between a fluorescent dye and the phosphor nano-particle, and varies depending on the type of the target substance and the type of the fluorescent dye. Those skilled in the art easily select a method for the labeling of a target substance.
When plural fluorescent dyes are used simultaneously, the dyes selected preferably emit fluorescence of different wavelengths from one another, from the viewpoint of easy detection of target substances by simultaneously measuring fluorescence. More preferably, the peaks for fluorescence emitted are apart from each other by 20 to 250 nm, and particularly preferably by 40 to 150 nm.
Furthermore, the fluorescent dyes to be used are preferably measurable each other by the order of energy transfer. Examples of such combinations include Alexa 488, 546, 594, and 647. In addition, similar measurement is possible by selecting a second fluorescent dye which has an absorption at a wavelength 15 nm or longer than the fluorescence spectrum maximum of the fluorescent dye having the shortest wavelength. Third and fourth dyes can be also selected in the same manner.

### [5] Linker

The phosphor nano-particle and fluorescent dyes, or fluorescent dyes and target substances in the invention are capable of binding (linking) each other, and the phosphor nano-particle and fluorescent dyes, and the fluorescent dyes and target substances preferably each have linkers which are capable of binding to each other.

Such linkers may be present as a part of the surface of the phosphor nano-particle and/or the fluorescent dyes, or directly or indirectly provided on the surface of the phosphor nano-particle and/or fluorescent dyes. The method for providing such linkers can be appropriately selected from means available to those skilled in the art. For example, a functional group such as an amino group or hydroxy group in the target substance or phosphor nano-particle may be directly bound to a reactive substituent such as a carboxyl group or active ester group in the fluorescent dye through an ionic bond or covalent bond; or a portion of a target substance may be subjected to a chemical modification such as introduction of a linker, followed by reaction with a fluorescent dye. The labeled substance after reaction can be purified by a common separation technique such as chromatography, electrophoresis, and recrystallization.
In the invention, the phosphor nano-particle and fluorescent dyes is preferably bound together via the surface of the above-described surface modifying agent because both dispersibility of the nanoparticle and the binding of the fluorescent dyes can be simultaneously realized. Example thereof include binding via a molecule binding to a reactive group such as an amino group or carboxyl group at the terminal of a surface modifying agent through amidation reaction or the like.

The linkers may be a pair of functional molecules which directly or indirectly bind to the surface of the phosphor nano-particle and/or fluorescent dyes, and examples thereof include nucleic acids which are complementary to each other (for example, monomer and oligonucleotide), antigen and antibody (monoclonal and polyclonal), and other affinity molecules such as protein (amino acid) and polysaccharides, for example, enzyme and substrate, avidin-biotin and the like.

Binding between functional molecules through amidation reaction is conducted by the condensation of a carboxyl group or a derivative group thereof (for example, ester, acid anhydride, and acid halide) and an amino group. When an acid anhydride or acid halide is used, it is preferably to use a base in combination. When an ester such as a methyl ester or ethyl ester of carboxylic acid is used, it is preferable to conduct heating or decompression to remove generated alcohol. When a carboxyl group is directly amidated, a substance for promoting amidation reaction may be added or reacted in advance, examples thereof include an amidation reagent such as DCC, Morpho-CDI, and WSC, a condensation additive such as HBT, or an active ester agent, such as N-hydroxy phthalimido, p-nitrophenyl trifluoroacetate, and 2,4,5-trichlorophenol. Furthermore, in the amidation reaction, either the amino group or carboxyl group of the affinity molecule to be bound through amidation is preferably protected with an appropriate protecting group according to a common procedure, and deprotected after the reaction.

The fluorescent composition of the invention may comprise a dispersion medium for dispersing the above-described phosphor nano-particle and fluorescent dyes. Examples of the dispersion medium include water and hydrophilic solvents, preferably methanol, ethanol, isopropanol, 2-ethoxyethanol and the like. The concentration of the phosphor nano-particle in the composition is not particularly limited because it varies with the fluorescence intensity, but is preferably 10⁻¹ M to 10⁻¹⁵ M, and more preferably 10⁻² M to 10⁻¹⁰ M. The concentration of the fluorescent dyes in the composition is not particularly limited because it varies with the type and fluorescence strength of the fluorescent dyes, but in general preferably 10⁻¹ M to 10⁻¹⁵ M, and more preferably 10⁻² M to 10⁻¹² M. These fluorescent compositions may be used in combinations when used with the phosphor nano-particle and fluorescent dyes.

The fluorescence detection method of the invention is a method for detecting two or more target substances contained in one sample by using the above-described phosphor nano-particle and the above-described fluorescent dyes. The detection method includes: forming a complex in which two or more fluorescent dyes, a phosphor nano-particle, and target substances are bound together; irradiating an excitation light for exciting the phosphor nano-particle to cause the phosphor nano-particle to emit light; exciting the above-described two or more fluorescent dyes bound to the above-described phosphor nano-particle with light emitted from the phosphor nano-particle to cause the fluorescent dyes to emit fluorescence; and detecting fluorescence emitted from the fluorescent dyes.
Since the fluorescent dyes bind to respective target substances, two or more fluorescent dyes are used according to the number of the target substances. Accordingly, plural target substances can be simultaneously detected by irradiating a single excitation light which excites the phosphor nano-particle.

In measurement of fluorescence emitted from the fluorescent dyes, the above-described labeling process and binding process may be performed in any order as long as the target substances, fluorescent dyes and phosphor nano-particle are bound to each other to form a complex. Accordingly, the phosphor nano-particle may be bound to the combination of the target substances and fluorescent dyes (a complex is formed after the target substances are labeled), or the target substances may be bound to the combination of the fluorescent dyes and phosphor nano-particle (the labeling is carried out after binding the phosphor nano-particle and fluorescent dyes). Emission of the phosphor nano-particle is usually performed after the formation of the complex, but may be performed before the formation of the complex. The order can be readily selected by those skilled in the art according to the type of the target substances, measurement conditions, or the like.

The target substances in the labeled sample may be dispersed in a liquid or fixed on a solid phase. The target substances to be detected may be any substance, but the target substances are preferably biological substances or compounds capable of interacting with biological substances, considering nanoparticles smaller than biological substances, and that do not affect the action of the biological substances.

Examples of such biological substances include hormones, tumor markers, enzymes, antibodies, antigens, abzymes, medicines, other proteins, nucleic acids, DNA, cDNA, and RNA. Examples of solid phases for immobilizing target substances thereon include gel supports such as agarose, membranes such as nitrocellulose and nylon, and glass. Immobilization of target substance on the solid phase is well known to those skilled in the art, and can be readily performed.

In the formation of a complex, the fluorescent dyes in a labeled sample bind to the phosphor nano-particle, thus energy is transferred to the fluorescent dyes from the phosphor nano-particle which emits light by the emission of the phosphor nano-particle, by which the fluorescent dyes are excited.
The excitation of the phosphor nano-particle is preferably performed by UV light from the viewpoint of detecting signal fluorescence in the visible range. Since a single type of the phosphor nano-particle is used, the irradiated UV light only has to have a wavelength which excites the phosphor nano-particle. The conditions for the excitation light is as described above.
The fluorescent dyes excited through the emission of the phosphor nano-particle preferably emit visible fluorescence light from the viewpoints of separation of excitation light and signal fluorescence, utilization of an inexpensive light source, and construction of a simple detection system. The conditions for the emission of the fluorescent dyes are as described above.

In the detection method according to the invention, fluorescence image detection systems in which fluorescence substances are used as target substances can be applied as they are.
In such systems, electrophoresed DNA fragments are labeled by: adding two or more types of corresponding fluorescent dyes to a solution containing plural of DNA fragments to be electrophoresed, followed by electrophoresis of the plural DNA fragments on a gel support. Or, plural DNA fragments are electrophoresed on a gel support containing the fluorescent dyes. Or plural DNA fragments are electrophoresed on a gel support, followed by immersion of the gel support in a solution containing the fluorescent dyes or the like.

Alternatively, plural DNA fragments are electrophoresed on a gel support, and the DNA fragments are denatured, subsequently at least a portion of the denatured DNA fragments is transferred onto a transfer support such as nitro cellulose by Southern blotting method. Then, the denatured DNA fragments are hybridized with a probe prepared by labeling DNA or RNA complementary to two or more target DNAs with two or more types each of fluorescent dyes, by which the DNA fragments complementary to the probe DNA or probe RNA are selectively labeled.

Further, a DNA probe complementary to the DNA containing target genes labeled with plural types of labeling agents is prepared, and hybridized with the DNA on a transfer support to combine enzymes with the complementary DNA labeled with the labeling agents, subsequently brought into contact with plural types of corresponding fluorescence substrates to change the fluorescence substrates to fluorescent dyes which emit light at different wavelengths.

In the above-described cases, two or more target substance are labeled with two or more fluorescent dyes. Therefore, after they are combined with the phosphor nano-particle to form a complex, plural target substances can be easily fluorescence-detected by exciting the phosphor nano-particle. For the fluorescence detection, a conventional method in which substances are detected by fluorescence of fluorescent dyes or the like can be applied as it is.
It is possible to generate an image by detecting fluorescence, by which the distribution of DNA on a gel support or a transfer support can be detected. The fluorescence image detection system has an advantage that gene sequences or the like can be simply detected without using radioactive substances.

Furthermore, the fluorescence detection method of the invention can be applied to a microarray image detection system as it is. In the system, a specific binding substance (for example, hormones, tumor markers, enzymes, antibodies, antigens, abzymes, other proteins, nucleic acid, cDNA, DNA, and RNA) which is capable of specifically binding to a substance from an organism, and is known about its base sequence, base length, composition and the like is dropped by using a spotter to form multiple independent spots in different locations on the surface of a carrier such as slide glass plate or membrane filter. Subsequently, the phosphor nano-particle is combined with a microarray hybridized with a substance from an organism (for example, hormones, tumor marker, enzyme, antibody, antigen, abzyme, other proteins, nucleic acid, cDNA, DNA, and mRNA), which has been collected from an organism by extraction, isolation, or the like and as necessary subjected to chemical treatment, chemical modification, or other treatment, and is labeled with two or more fluorescent dyes, and then an excitation light is irradiated to the phosphor nano-particle to photoelectrically detect the fluorescence emitted from the fluorescent dyes for analyzing the substance derived from an organism. The microarray image detection system has an advantage that it can analyze a substance derived from an organism in a short period of time by forming multiple spots of a specific binding substance at different locations in a dense pattern on the surface of a carrier such as a slide glass plate, and hybridizing them with the substance derived from organisms labeled with a labeling agent.

Furthermore, the present detection method can be also used for the analysis of an intermolecular interaction in a solution system. For example, intermolecular interaction or specific binding between biomolecules can be analyzed using FRET (Fluorescence Resonance Energy Transfer). By measuring the state of plural molecules such as proximity, coupling, or dissociation using fluorescence, the interaction between plural molecules can be observed successively.

The invention is further illustrated by following Examples, but the invention is not limited to them.

### Example 1

### [Synthesis of surface-modified metal oxide phosphor nano-particles]

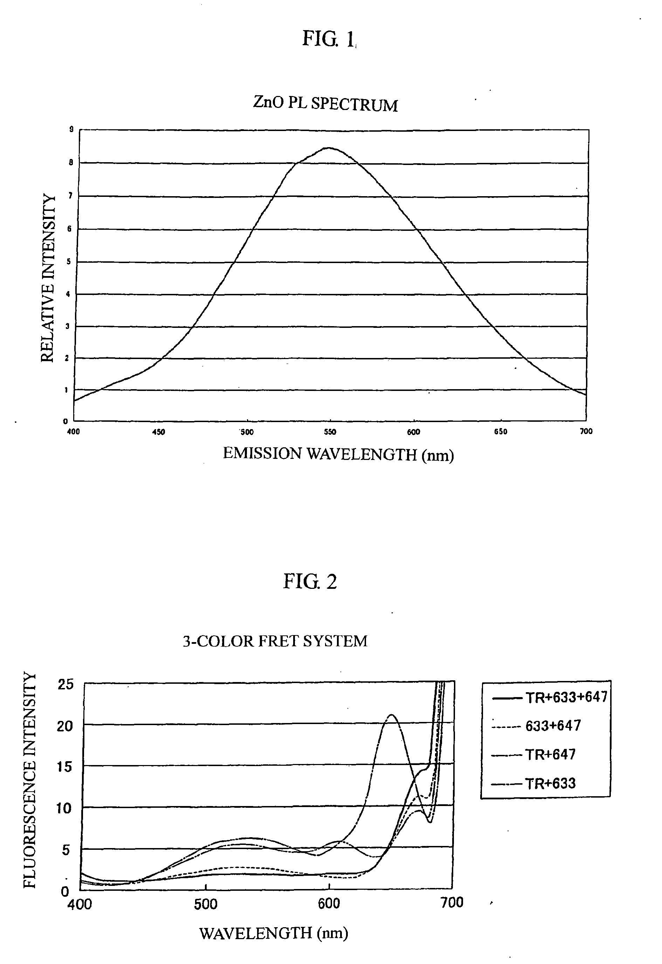

8.8 g of zinc acetate dihydrate was dissolved in 400 ml of dehydrated ethanol, and 240ml of the solution was removed by evaporation under reflux at 93°C for 2 hours. 240 ml of dehydrated ethanol was added, and cooled to room temperature. 18 ml of 25% by mass tetramethylammonium hydroxide solution in methanol was added, and stirred for 30 minutes. 7.2 ml of 3-aminopropyltrimethoxysilane and 2.2 ml of water were added, and stirred at 60°C for 4 hours. White sedimentation obtained was removed by filtration, washed with ethanol, and then dried.
By XRD and TEM analyses, the precipitate was found to be ZnO nanoparticles having an average particle diameter of approximately 4 nm. Furthermore, binding of Si and aminopropyl groups to the surface of the ZnO particles was confirmed by elementary analysis, and IR absorption spectroscopy. Water was added to the precipitate to make a 2% by mass aqueous dispersion liquid. Fig. 1 shows the fluorescence spectrum of the dispersion liquid irradiated with a light of 370 nm. A strong fluorescence having a peak wavelength of 540 nm and a half width of 145 nm was exhibited.

### Example 2

### [Biotinylation reaction of zinc oxide nanoparticle (ZnO)]

1.25 ml of 1.4% surface-aminated ZnO solution prepared in Example 1 was combined with 1.25 ml of 0.1 M HEPES buffer solution (pH 8.0), and with stirred at room temperature, 30 µg of a biotinylating reagent (Biotin-(AC5)2Sulfo-OSu; Dojindo Laboratories) was added, and reacted for 2 hours. After the completion of the reaction, the reaction liquid was purified by gel chromatography (trade name: PD-10 column; Amersham Biosciences Co.) to obtain 3.5 ml of a biotinylated ZnO solution.

### Example 3

### [Fluorescence measurement]

The fluorescent dyes used are as follows.
Streptavidin-conjugated Texas Red [manufactured by Invitrogen Japan K.K.] Excitation wavelength 597 nm, fluorescence wavelength 616 nm
Streptavidin-conjugated Alexa 633 [manufactured by Invitrogen Japan K.K.] Excitation wavelength 637nm, fluorescence wavelength 651 nm
Streptavidin-conjugated Alexa 647 [manufactured by Invitrogen Japan K.K.] Excitation wavelength 652 nm, fluorescence wavelength 671 nm
All the above dies were stocks dissolved to a concentration of 1 mg/ml.
The measurement of fluorescence was performed at an excitation wavelength of 350 nm using a fluorescence spectrophotometer (trade name: FP-750, manufactured by JASCO Corporation), and a fluorescence spectrum at 400 to 700 nm was measured.

### Example 4

### [Multi-color system fluorescence measurement]

The mixture liquid of the fluorescent dyes in Example 3 was added with biotinylated ZnO, and the change in the fluorescence spectrum was measured. Three different measurements were performed using a combination of the three, and the three ways of combining any two of the fluorescent dyes. The procedure is as follows.

| | |
|---|---|
| 0.1 M HEPES buffer (pH 8.0) | 2.25ml |
| Solutions of streptavidin-conjugated dyes | 5 µl each |

The above solutions were added with 0.25 ml of biotinylated ZnO, and immediately subjected to measurement. The results are shown in Fig. 2.

When the combination of the three dyes was used, only Alexa 647-derived fluorescence was observed, other than the ZnO-specific fluorescence (540 nm). Furthermore, when the combination of Alexa 633 and 647 was used, only the Alexa 647-derived fluorescence was observed, and when the combination of Texas Red and Alexa 633 was used, only the Alexa 633-derived fluorescence was observed. On the other hand, when the combination of Texas Red and Aiexa 647 was used, the fluorescence derived from both of them was observed. From the above observations, an energy transfer system as shown in the following Table 1 can be supposed.

**[Table 1]**

| a) Combination of three dyes |
|---|
| Excitation light ⇒ ZnO ⇒ Texas Red ⇒ Alexa 633 ⇒ Alexa 647 |

| b) Combination of two dyes |
|---|
| Excitation light → ZnO → Alexa 633 → Alexa 647 |
| Excitation light → ZnO → Texas Red → Alexa 633 |
| Excitation light → ZnO → Texas Red ↓ Alexa 647 |

As shown in Table 1, when a fluorescent dye having an excitation wavelength in the broad fluorescence wavelength range of ZnO is in proximity to a ZnO nanoparticle through the linker of avidin-biotin or the like, it is readily subjected to energy transfer to emit fluorescence. Furthermore, when fluorescent dyes having overlapped excitation and fluorescence wavelength ranges were combined, it was shown that energy transfer between dyes is possible.
Accordingly, the combination of ZnO nanoparticle and two or more fluorescent dyes allows the construction of a system for obtaining optional fluorescence in the visible range using a single UV excitation light.
With such a system, plural target substances can be simply, efficiently, and simultaneously detected.

## Claims

1. A fluorescent material comprising: two or more fluorescent dyes that emit light at wavelengths different from each other; and a phosphor nano-particle bound to each of the two or more fluorescent dyes.

2. The fluorescent material of Claim 1 wherein the phosphor nano-particle is capable of transferring energy to the fluorescent dyes at energy levels different from each other.

3. The fluorescent material of Claim 1 or 2 wherein the half width of the emission of the phosphor nano-particle is 50 to 200 nm.

4. The fluorescent material of any one of Claims 1 to 3 wherein each of the phosphor nano-particle and the fluorescent dyes have linkers capable of binding the nanoparticle phosphor and the fluorescent dyes to each other.

5. The fluorescent material of any one of Claims 1 to 4 wherein the phosphor nano-particle is a surface-modified phosphor nano-particle of a metal oxide, or a surface-modified nanoparticle phosphor of a metal sulfide, that is surface-modified with a surface modifying agent of a compound represented by the following formula (I) or a decomposition product thereof:
M-(R)₄ (I)
wherein in formula (I), M represents a Si or Ti atom, and R represents an organic group, each R group may be the same or different from each other, but at least one R group is a group having reactivity with an affinity molecule.

6. A fluorescent composition comprising: two or more fluorescent dyes which emit light at wavelengths different from each other; and a phosphor nano-particle capable of binding to each of the two or more fluorescent dyes.

7. A fluorescence detection method for detection of two or more target substances contained in one sample by using two or more fluorescent dyes, each of the fluorescent dyes being capable of binding to the respective target substance and each of the fluorescent dyes emitting light at a wavelength different from each other, the method comprising:
forming a complex in which a phosphor nano-particle capable of binding to each of the two or more fluorescent dyes, the fluorescent dyes, and the target substances are bound together;
irradiating an excitation light for exciting the phosphor nano-particle to cause the phosphor nano-particle to emit light;
exciting the two or more fluorescent dyes bound to the phosphor nano-particle with light emitted from the phosphor nano-particle to cause the fluorescent dyes to emit fluorescence; and
detecting fluorescence emitted from the fluorescent dyes.
